# EUROPÄISCHE PATENTSCHRIFT

(11) **EP 0 428 875 B1**
(45) Veröffentlichungstag und Bekanntmachung des Hinweises auf die Patenterteilung: **26.10.1994**
(21) Anmeldenummer: 90120190.5
(22) Anmeldetag: 20.10.1990
(51) Int. Cl.: A61F 7/12

(54) **Einrichtung zur Behandlung von Erkrankungen der Wände von Körperöffnungen bzw. Körperhöhlen**
Apparatus to treat diseases of the walls of body orifices or cavities
Appareil de traitement des maladies de parois d'orifices ou de cavités du corps

(30) Priorität: 24.10.1989 CH 3836/89
(43) Veröffentlichungstag der Anmeldung: 29.05.1991
(73) Patentinhaber: ZEWA AG, CH-6052 Hergiswil (CH)
(72) Erfinder: Zeindler, Kurt, CH-6030 Ebikon (CH)
(74) Vertreter: Scheidegger, Werner & Co.

(56) Entgegenhaltungen:
- EP-A- 0 330 472
- WO-A-89/04137
- FR-A- 774 550
- FR-A- 2 600 205
- US-A- 1 964 732
- US-A- 4 240 441
- US-A- 4 411 648
- US-A- 4 585 002

## Beschreibung

Die vorliegende Erfindung betrifft gemäß dem Obergriff des Anspruchs 1 eine Einrichtung zur Behandlung von Erkrankungen der Wände von Körperöffnungen des menschlichen oder tierischen Körpers, insbesondere der Wände des Darmendtraktes oder des Harnleiters, mit einer in die Körperöffnung einführbaren Sonde, welche mit einer elektrischen Heizvorrichtung ausgerüstet ist, um über die Sondenwand Wärme an die Wand der Körperöffnung abzugeben, ferner mit einem Steuergerät, welches eine elektrische Energiequelle enthält, bzw. an eine solche anschliessbar ist, um elektrische Energie an die Heizvorrichtung der Sonde abzugeben.

Einrichtungen dieser Art sind bekannt (siehe z.B. US-A-1 964 732) und im Handel erhältlich. Sie wurden insbesondere für die Wärmebehandlung von Hämorrhoiden entwickelt und werden vom Arzt in der Privatpraxis sowie in Kliniken und Spitälern sowie auch zur Heimtherapie verwendet. Die Geräte haben sich bewährt und klinische Tests haben gezeigt, dass damit die Blutzirkulation im Analbereich gefördert und dadurch die Grösse der Hämorrhoiden verringert wird, dies ohne zusätzlichen Einsatz von Medikamenten. Die Tests haben zudem gezeigt, dass eine Behandlung mittels solcher Einrichtungen vom Patienten und dies im Gegensatz zu anderen Therapieformen als relativ angenehm empfunden wird.

Aehnliche bekannte Einrichtungen bestehen, neben der eigentlichen Sonde, aus einem Kontroll- und Steuergerät, welches u.a. mit einer Temperaturanzeige, einem Uhrwerk mit Anzeige (aktuelle Zeit sowie einer rückwärts zählenden Vorgabezeit) und den notwendigen Schaltern, z.B. Start/Stop/Pause, ausgerüstet ist. Das Gerät enthält einen Steuerstromkreis, welcher erlaubt, die Sonde während der Behandlung zu speisen und zu kontrollieren. Geeignete Schaltungen können von jedem Fachmann der Elektrotechnik bzw. Elektronik gebaut werden. Die gewünschte Temperatur ist auf einen Wert von z.B. zwischen 37-46°C einstellbar, wobei die Schaltung dafür besorgt ist, dass ein maximal eingestellter Wert eingehalten wird (die Sonde ist mit einem entsprechenden Temperaturfühler ausgerüstet). Das Gerät ist mit allen notwendigen Sicherheits-Systemen ausgerüstet:
So muss das Gerät z.B. über ein eingebautes Ladegerät aus dem Netz aufgeladen werden. Das Gerät kann nicht eingesetzt werden, solange es an das Netz angeschlossen ist. Während der eigentlichen Behandlung wird das Gerät aus den eingebauten Akkus gespeist und erzeugt in der Sonde die gewünschte Wärme.

Der Aufbau einer Behandlungseinrichtung ist z.B. in der CH-PS 618 875 beschrieben. Ferner wird Wirkung und Einsatz eines solchen Gerätes zusammen mit klinischen Resultaten in der "Schweizerischen Rundschau für Medizin (Praxis) 76, Nr. 49" aus dem Jahr 1987 beschrieben.

Obwohl an sich befriedigende Resultate mit den bekannten Geräten erzielt werden konnten, wurde nach Möglichkeiten gesucht, die Wirkung der Behandlung zu verbessern und diese gleichzeitig auf die Behandlung von Erkrankungen der Wände anderer Körperöffnungen, insbesondere der Wände des Harnleiters (Prostata) anzuwenden.

Bei Behandlungsgeräten für äussere Hautoberflächen wurde ein Gerät geschaffen, dessen Kontaktfläche intermittierend gekühlt bzw. aufgeheizt wird. Dies wird mittels Peltier-Elementen realisiert, wobei die beim Kühlen gleichzeitig anfallende Wärme über Kollektorlamellen an die Umgebungsluft abgeführt wird. Ein solches Gerät ist in US-A-4,585,002 beschrieben und erlaubt eine Behandlung "kalt/warm", wäre jedoch in einem Gerät nach US-A-1,964,732 zum Einführen in Körperhöhlen nicht anwendbar.

Versuche haben nun aber gezeigt, dass bei der Behandlung von Körperhöhlen durch intermittierende Wärmepulse die Resultate wesentlich verbessert werden konnten. Zu diesem Zweck wurde die Behandlungseinrichtung erfindungsgemäss ausgerüstet.

Aufgabe der vorliegenden Erfindung war die Schaffung einer Einrichtung der eingangs definierten Art, welche es erlaubt, die Therapieergebnisse zu verbessern.

Die Einrichtung zeichnet sich zur Lösung dieser Aufgabe erfindungsgemäss dadurch aus, dass im Stromkreis für die Heizvorrichtung Mittel vorgesehen sind, um die Heizvorrichtung derart mit Strom zu versorgen, dass diese die Wärme in Form von sich zeitlich folgenden, bzw. intermittierenden Wärmeimpulsen einstellbarer Grösse abgibt.

Eine bevorzugte Ausführungsform zeichnet sich dadurch aus, dass die Mittel im Steuerstromkreis der Heizvorrichtung eine Schaltanordnung umfassen, welche die Stromzufuhr zur Heizvorrichtung periodisch für eine vorbestimmte Zeit mindestens reduziert, um so die Wärme in Form von Wärmeimpulsen abzugeben.

Vorzugsweise ist die Einrichtung derart ausgestaltet, dass im Steuerstromkreis der Heizvorrichtung zusätzlich Mittel vorgesehen sind, um die durch die Heizvorrichtung erzeugte Temperatur zu überwachen und dass vorzugsweise Mittel vorgesehen sind, um, gesteuert durch die zusätzlichen Mittel, diese Temperatur zu begrenzen.

Ueblicherweise besteht die elektrische Heizvorrichtung aus einem elektrischen Heizwiderstand.

Zweckmässigerweise ist zur Erfassung und vorzugsweise zur Regelung der Temperatur des die erzeugte Wärme nach aussen übertragenden Sondenwandabschnittes ein Sensor vorgesehen.

Zu diesem Zweck zeichnet sich die Einrichtung der vorstehend beschriebenen Bauart, bei welcher die Sonde in Form eines zylindrischen Körpers mit geringem Durchmesser ausgebildet ist, erfindungsgemäss dadurch aus, dass sie zusätzlich einen Ballonkatheter umfasst, durch welchen nach dessen Aufblasen die Sonde in eine Körperöffnung mit geringer Weite, z.B. in eine Harnröhre, bzw. einen Harnleiter einführbar ist. Dadurch ist eine genaue Plazierung der Sonde erst möglich.

Selbstverständlich können mehrere Sonden, z.B. mit verschiedenen Formen und Durchmessern, vorgesehen sein, welche wahlweise an das Steuergerät anschliessbar sind. Für den Darmeinsatz, ohne Katheter, weist die zylindrische Sondenform mindestens eine kragenförmig aufragende Sperrpartie auf, um ein unkontrolliertes Einwärtsgleiten zu verhindern. Für den Harnleitereinsatz ist sie von kleinerem Durchmesser und zylindrisch.

Eine weitere Verbesserung kann dadurch erzielt werden, dass die Sonde als länglicher Körper ausgebildet ist, dessen mittlerer Wandabschnitt zur Wärmeübertragung nach aussen dient, während zu beiden Seiten dieses Wandabschnittes je ein elektrisch leitender, elektrisch vom andern getrennter Wandabschnitt vorgesehen ist, die an unterschiedliche elektrische Potentiale anschliessbar sind, derart, dass im Betrieb über die anliegende Körperwand ein durch die Potentialdifferenz getriebener Strom fliessen kann.

Vorzugsweise ist eine solche Einrichtung so ausgebildet, dass der wärmeübertragende mittlere Wandabschnitt aus einem elektrisch nichtleitenden Material und die beidseitig benachbarten Wandabschnitte aus elektrisch leitendem Material bestehen.

Die Erfindung wird nachstehend anhand von in der Zeichnung dargestellten Beispielen noch näher erläutert. Es zeigt:
Fig. 1 eine schematische Gesamtdarstellung einer erfindungsgemässen Einrichtung;
Fig. 1a eine schematische Darstellung der elektrischen/elektronischen Schaltung;
Fig. 2 eine Sonde, schematisch, im Längsschnitt;
Fig. 3 eine andere Ausführungsform einer Sonde, mit mittlerem Heizabschnitt und zwei an unterschiedliche Potentiale anschliessbaren Endabschnitten, hier für Darmeinsatz, und
Fig. 4 eine aus Ballon-Katheter und Sonde bestehende Einrichtung.

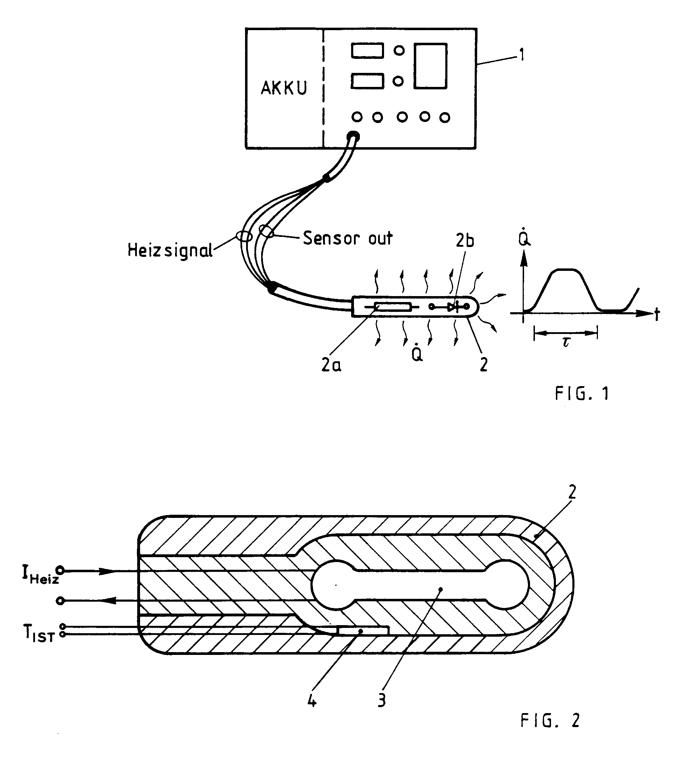

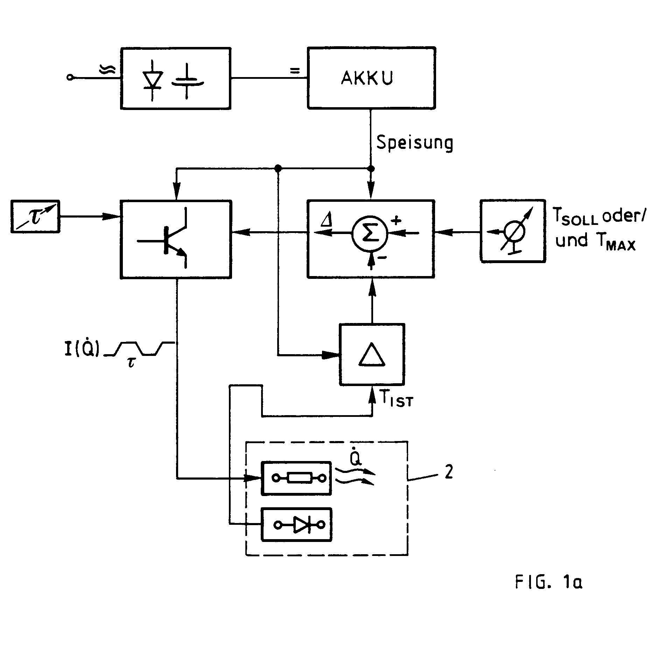

Fig. 1 der Zeichnung zeigt schematisch eine erfindungsgemässe Einrichtung, wie sie z.B. zur Wärmebehandlung von Hämorrhoiden oder, bei entsprechend ausgebildeter Sonde und gegebenenfalls zusammen mit einem Ballonkatheter, zur Wärmebehandlung von Prostata verwendet werden kann.

Die Einrichtung weist ein Steuergerät 1 auf, welches den gesamten elektrischen bzw. elektronischen Steuerstromkreis in Form einer passenden Schaltung enthält und mit verschiedenen Informations-Anzeigen versehen ist, so z.B. einer Temperaturanzeige, Uhrzeit, Uhrzeit als Vorgabezeit rückwärts zählend etc. sowie mit den notwendigen Schaltern bzw. Einstellknöpfen ausgerüstet ist. Einstellbar ist u.a. die maximale Behandlungstemperatur (einstellbar z.B. zwischen 37 - 50°C), die Dauer τ der intermittierenden Wärmeimpulse Q und die gewünschte Behandlungszeit (z.B. 20-30 Min.).

Ebenfalls enthält das Steuergerät 1 ein gegebenenfalls an das Netz anschliessbares Ladegerät und einen oder mehrere aufladbare Akkus.

Schliesslich ist die in die zu behandelnde Körperöffnung einzuführende Sonde 2 mit der entsprechenden Stromzuführung vorgesehen. Die Sonde 2 enthält neben einer elektrischen Heizvorrichtung 2a auch einen Sensor 2b, welcher zur Steuerung der gewünschten maximalen Temperatur dient.

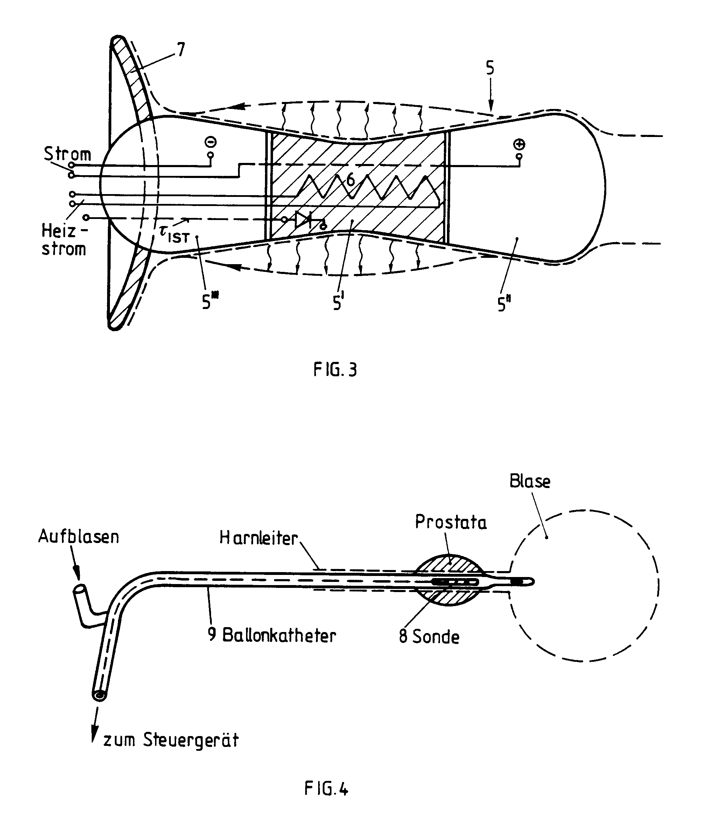

Fig. 2 zeigt rein schematisch eine solche Sonde 2, mit einer Heizvorrichtung in Form eines elektrischen Heizwiderstandes 3 und einem Sensor 4. Ausgezogen hat sie die Form als Katheter-Harnleitersonde, gestrichelt als Darmsonde (Fig. 3). Die Zuleitungen für den Heizwiderstand 3 und den Sensor 4 sind vorzugsweise am Ausgang der Sonde 2 in einem Kabel zusammengefasst, welches an das Steuergerät 1 anschliessbar ist. Aus Sicherheitsgründen (Vorschriften) sind die Geräteanschlüsse in bekannter Weise so ausgestaltet, dass bei ans Netz angeschlossenem Gerät nicht gleichzeitig die Sonde an dieses Gerät angeschlossen werden kann.

Im Steuerstromkreis für die Heizvorrichtung, siehe Schema gemäss Fig. 1a, sind Mittel vorgesehen, um die Stromzufuhr zur Heizvorrichtung intermittierend, z.B. periodisch, für eine vorbestimmte Zeit zu reduzieren, derart, dass die Heizvorrichtung die darin erzeugte Wärme Q in Form von sich zeitlich folgenden bzw. intermittierenden Wärmeimpulsen abgibt, wobei die maximale Temperatur Tmax der Wärmeimpulse einstellbar ist.

Fig. 3 der Zeichnung zeigt eine Variante einer Sonde 5 für Darmeinsatz. Diese besteht aus einem länglichen, zäpfchenartigen Grundkörper, in dessen mittlerem Wandabschnitt 5', durch die elektrische Heizvorrichtung 6, Wärme nach aussen abgegeben wird in Form von intermittierenden Wärmeimpulsen). Die beidseitig an den mittleren Wandabschnitt 5' anschliessenden Wandabschnitte 5'' und 5''' sind vom mittleren Wandabschnitt elektrisch isoliert und sie werden an unterschiedliche Potentiale + bzw. - angelegt, so dass im Betrieb über die (feuchte) Körperwand zusätzlich zur Wärme ein Strom durch das Gewebe fliessen kann, was zu einer weiteren Verbesserung der Therapiewirkung führt. Die Stromzufuhr zu den Wandabschnitten 5'', 5''' kann kontinuierlich oder ebenfalls intermittierend erfolgen. Im letzteren Fall kann die Stromzufuhr synchron mit jener zum Heizwiderstand 6 erfolgen. Vorzugsweise weist die Sonde 5 am einen Ende einen flanschartigen Anschlag 7 auf, um zu verhindern, dass die Sonde zu weit in die Körperöffnung eindringen kann.

Fig. 4 schliesslich zeigt eine erfindungsgemässe Einrichtung (ohne Steuergerät), welche neben der sehr gedrungenen Sonde 8 einen sog. Ballonkatheter 9 umfasst. Damit ist es möglich, die Sonde 8 auch in relative enge Körperöffnungen bzw. Körperhöhlen einzuführen und dort genau zu plazieren, wie z.B. in Harnleiter, um damit Prostataleiden zu behandeln. Die Funktionsweise des Ballonkatheters ist den Fachleuten geläufig.

## Patentansprüche

1. Einrichtung zur Behandlung von Erkrankungen der Wände von Körperöffnungen des menschlichen oder tierischen Körpers, insbesondere der Wände des Darmendtraktes oder des Harnleiters, mit einer in die Körperöffnung einführbaren Sonde (2), welche mit einer elektrischen Heizvorrichtung (3) ausgerüstet ist, um über die Sondenwand Wärme an die Wand der Körperöffnung abzugeben, ferner mit einem Steuergerät (1), welches eine elektrische Energiequelle enthält bzw. an eine solche anschliessbar ist, um elektrische Energie an die Heizvorrichtung (3) der Sonde (2) abzugeben, dadurch gekennzeichnet, dass im Steuerstromkreis für die Heizvorrichtung (3) Mittel vorgesehen sind, um die Heizvorrichtung(3) derart mit Strom (I) zu versorgen, dass diese die Wärme (Q) in Form von sich zeitlich folgenden bzw. intermittierenden Wärmeimpulsen einstellbarer Grösse (Q̇;τ) abgibt.

2. Einrichtung nach Anspruch 1, dadurch gekennzeichnet, dass die Mittel im Steuerstromkreis der Heizvorrichtung (3) eine Schaltanordnung umfassen, welche die Stromzufuhr (I) zur Heizvorrictung (3) periodisch für eine vorbestimmte Zeit (τ') mindestens reduziert, um so die Wärme (Q) in Form von Wärmeimpulsen (Q̇) abzugeben.

3. Einrichtung nach Anspruch 1 oder 2, dadurch gekennzeichnet, dass im Steuerstromkreis der Heizvorrichtung (3) zusätzlich Mittel (4) vorgesehen sind, um die durch die Heizvorrichtung (3) erzeugte Temperatur zu überwachen und dass vorzugsweise Mittel vorgesehen sind, um, gesteuert durch die zusätzlichen Mittel (4), diese Temperatur zu begrenzen.

4. Einrichtung nach einem der Ansprüche 1-3, dadurch gekennzeichnet, dass die elektrische Heizvorrichtung (3) eine elektrische Widerstandsheizung ist.

5. Einrichtung nach einem der Ansprüche 1-4, dadurch gekennzeichnet, dass zur Erfassung und vorzugsweise zur Regelung der Temperatur des die erzeugte Wärme (Q) nach aussen übertragenden Sondenwandabschnittes (2) ein Sensor (4) vorgesehen ist.

6. Einrichtung nach einem der Ansprüche 1-5, bei welcher die Sonde in Form eines zylindrischen Körpers (8) mit geringem Durchmesser ausgebildet ist, dadurch gekennzeichnet, dass sie zusätzlich einen Ballonkatheter (9) umfasst, durch welchen, nach dessen Aufblasen, die Sonde (8) in eine Körperöffnung mit geringer Weite, z.B. in eine Harnröhre bzw. einen Harnleiter einführbar ist.

7. Einrichtung nach einem der vorangehenden Ansprüche, dadurch gekennzeichnet, dass sie mehrere auswechselbare Sonden (2) aufweist, welche wahlweise an das Steuergerät (1) anschliessbar sind.

8. Einrichtung nach einem der Ansprüche 1-7, dadurch gekennzeichnet, dass die Sonde als länglicher Körper (5) ausgebildet ist, dessen mittlerer Wandabschnitt (5') zur Wärmeübertragung nach aussen dient, während zu beiden Seiten dieses Wandabschnittes zwei elektrisch leitende, elektrisch voneinander getrennte Wandabschnitte (5'', 5''') vorgesehen sind, welche an unterschiedliche elektrische Potentiale (+,-) anschliessbar sind, derart, dass im Betrieb über die anliegende Körperwand eine Potentialdifferenz anliegt.

9. Einrichtung nach Anspruch 8, dadurch gekennzeichnet dass der wärmeübertragende mittlere Wandabschnitt (5') aus einem elektrisch nichtleitenden Material und die beidseitig benachbarten Wandabschnitte (5'', 5''') aus elektrisch leitendem Material bestehen.

10. Einrichtung nach einem der Ansprüche 8 oder 9, dadurch gekennzeichnet, dass am einen Ende des länglichen Sondenkörpers (5) ein flanschartiger, radial nach aussen abstehender Anschlag (7) vorgesehen ist.

## Claims

1. An apparatus for the treatment of diseases of the walls of openings of a human or animal body, specifically of the walls of the terminal tract of the bowels or of the ureter, including a probe (2) adapted to be inserted into a respective opening of the body which probe has an electrical heating unit (3) adapted to deliver heat to the wall of the opening of the body via a wall of the probe, including further a control apparatus (1) which contains a source of electrical energy or is connectable to such source, such to transfer electrical energy to said heating unit (3) of said probe (2), characterized by a control circuit for said heating unit (3) which includes means for supplying said heating unit (3) such with electrical current (I) that latter emits the heat (Q) in form of chronological consecutive or intermittent heat impulses of an adjustable magnitude (Q̇;τ).

2. The apparatus of claim 1, characterized in that said supplying means in said control circuit of the heating unit (3) comprise a circuitry adapted to at least reduce the supply of current (I) to said heating unit (3) periodically for a predetermined time span (τ') in order to emit the heat (Q) in form of heat impulses (Q̇).

3. The apparatus of claim 1 or 2, characterized in that said control circuit for said heating unit (3) comprises further means (4) for controlling the temperature generated by said heating unit (3) and comprises preferably means controlled by said further controlling means (4) for limiting said temperature.

4. The apparatus of any of claims 1-3, characterized in that said electrical heating unit (3) is an electrical resistance heater.

5. The apparatus of any of claims 1-4, characterized by a sensor (4) adapted to detect and preferably control the temperature of the wall of said probe (2) which transmits the heat (Q) generated towards the outside.

6. The apparatus of any of claims 1-5, wherein said probe has the shape of a cylindrical body (8) having a small diameter, characterized in that said probe comprises further a balloon catheter (9) by means of which after an inflating thereof said probe (8) is insertable into an opening of a body having a small width, e.g. into a urethra or ureter.

7. The apparatus of any of the foregoing claims, characterized in that it comprises a plurality of exchangeable probes (2) which are selectively connectable to said control apparatus (1).

8. The apparatus of any of claims 1-7, characterized in that said probe has the shape of an elongate body (5) of which the center wall portion (5') is adapted to transmit heat towards the outside and wherein at both sides of said center wall portion electrically conducting wall portions (5'', 5''') are located which are electrically separated from each other and are connectable to differing electrical potentials (+, -) whereby in operation a potential difference is present over the contacting wall of the body.

9. The apparatus of claim 8, characterized in that said center heat transmitting wall portion (5') consists of an electrically nonconductive material and the at both sides thereof adjacent wall portions (5'', 5''') consist of an electrically conductive material.

10. The apparatus of claim 8 or 9, characterized in that a flange-like radially projecting abutment (7) is located at one end of said elongate probe body (5).

## Revendications

1. Appareil de traitement des maladies des parois d'orifices du corps humain ou animal, notamment des parois du tract terminal intéstinal ou de l'uretère, comprenant une sonde (2) pouvant être introduite dans ledit orifice du corps et équipée d'un dispositif de chauffage électrique (3) pour fournir de la chaleur à la paroi de l'orifice du corps à travers la paroi de la sonde, et un dispositif de commande (1) contenant une source d'énergie électrique ou pouvant respectivement être reliée à une telle source pour fournir de l'énergie électrique au dispositif de chauffage (3) de la sonde (2), **caractérisé** en ce que le circuit du courant de commande pour le dispositif de chauffage (3) comprend des moyens pour alimenter le dispositif de chauffage (3) avec du courant (I) d'une telle manière que le dispositif de chauffage fournit la chaleur (Q) sous forme d'impulsions de chaleur chronologiques consécutives ou intermittentes de grandeur réglable (Q̇,τ).

2. Appareil selon la revendication 1, caractérisé en ce que lesdits moyens dans le circuit de commande du dispositif de chauffage (3) comprennent un circuit qui réduit au moins périodiquement pendant un certain temps (τ') l'amenée de courant (I) au dispositif de chauffage (3) pour ainsi délivrer la chaleur (Q) sous forme d'impulsions de chaleur (Q̇).

3. Appareil selon l'une des revendications 1 ou 2, caractérisé en ce que le circuit de commande du dispositif de chauffage comprend des moyens additionnels (4) pour contrôler la température produite par le dispositif de chauffage (3) et de préférence des moyens, commandés par lesdits moyens additionnels (4), pour limiter cette température.

4. Appareil selon l'une des revendications 1-3, caractérisé en ce que le dispositif de chauffage électrique (3) est un chauffage à résistance ohmique.

5. Appareil selon l'une des revendications 1-4, caractérisé par un détecteur (4) pour détecter et de préférence pour régler la température de la section de la paroi de la sonde (2) qui transmet la chaleur (Q) vers l'extérieur.

6. Appareil selon l'une des revendications 1-5, dans lequel la sonde présente la forme d'un corps cylindrique (8) à faible diamètre, caractérisé en ce qu'il comprend en outre un cathéter à ballon (9), à travers lequel, après inflation, la sonde (8) peut être introduite dans un orifice du corps de petit diamètre, par exemple dans un uretère.

7. Appareil selon l'une des revendications précédentes, caractérisé en ce qu'il présente plusieurs sondes (2) échangeables qui peuvent être raccordées à volonté au dispositif de commande (1).

8. Appareil selon l'une des revendications 1-7, caractérisé en ce que la sonde se présente sous forme d'un corps longitudinal (5) dont la section de paroi médiane (5') sert à la transmission de la chaleur vers l'extérieur et qui, de chaque côté de cette section de paroi, présente des sections de paroi (5'', 5''') conductrices d'électricité mais électriquement isolées l'une de l'autre pouvant être raccordées à des potentiels électriques différents (+, -) de manière à ce qu'en opération une différence de potentiel se présente sur la paroi de corps en contact.

9. Appareils selon la revendication 8, caractérisé en ce que la section de paroi médiane (5') transférant la chaleur est fait d'un matériel isolant tandis que les deux sections de paroi (5'', 5''') adjacentes sont en un matériel électriquement conducteur.

10. Appareils selon l'une des revendications 8 ou 9, caractérisé en ce qu'une des extrémités du corps de sonde (5) longitudinal présente une butée (7) sous forme d'un flange s'étendant radialement vers l'extérieur.
